# EUROPEAN PATENT APPLICATION

(11) **EP 4 796 108 A1**
(43) Date of publication of application: **26.08.2026**
(21) Application number: 23942214.0
(22) Date of filing: 20.06.2023
(51) Int. Cl.: A61K 9/127, A61K 38/16, C07K 14/00

(54) **FUNCTIONALISED LIPOSOMES AND USE THEREOF TO PRODUCE COSMETIC OR PHARMACEUTICAL COMPOSITIONS FOR THE TREATMENT OF PSORIASIS AND VITILIGO**

(71) Applicant: Dermopartners, S.L., 46138 Rafelbunyol - Valencia (ES)
(72) Inventor: SERRANO SANMIGUEL, Gabriel, 46138 Rafelbunyol - Valencia (ES); SERRANO BALLESTEROS, Pilar, 46138 Rafelbunyol - Valencia (ES); SERRANO NUÑEZ, Juan Manuel, 46138 Rafelbunyol - Valencia (ES); TORRENS MARTINEZ, Ana, 46138 Rafelbunyol - Valencia (ES)
(74) Representative: Urizar Anasagasti, Jesus Maria
(86) International application number: PCT/ES2023/070402
(87) International publication number: WO 2024/261352

(57) **Abstract**

The invention relates to liposomes functionalized with a peptide with affinity for the receptor CXCR3-B, and to the use of same to obtain pharmaceutical or cosmetic preparations for the treatment of psoriasis and vitiligo.

## Description

### Technology sector

The object of the present invention has application in the field of the medical, pharmaceutical and cosmetic industries, since it concerns liposomes and their use in the preparation of compositions that are used to treat skin alterations in cases of psoriasis and vitiligo.

### Background of the invention

Among the prior art of this invention, we can find patent DE4021084, which refers to a "Topical fumarate compound containing liposomes that improve bioavailability, especially for the treatment of psoriasis. These liposomes contain a liposomal structure, alcohol, a stabilizer, and fumaric acid (derivative), and are stable and nonirritating." However, in this case, liposomes functionalized with a peptide that has affinity for cell receptors overexpressed in cases of psoriasis are not used.

Another relevant precedent is US patent 2013053319 (A1) concerning "Chemokine-derived peptides and uses for chronic wound and angiogenesis inhibition treatments," which describes peptides with activity against the CXCR3 receptor. The present invention relates to peptides with angiogenesis-inhibiting activity that may be useful in the treatment of diseases associated with excessive angiogenesis, including tumorigenic diseases such as cancers, genes encoding these peptides, and methods and uses thereof in the treatment of such diseases. In this case, the use of these peptides is for their direct effect in the treatment of these diseases, unlike the present invention, in which the peptides are used to bind to liposomes containing certain active ingredients and enhance the effect of these active ingredients in cases of psoriasis and vitiligo.

### Description of the invention

The present invention relates to liposomes functionalized with a peptide having affinity for the CXCR3B receptor and their use in pharmaceutical or cosmetic preparations for the topical treatment of psoriasis and vitiligo.

Liposomes are nanometric vesicles. They are capable of encapsulating both hydrophilic and hydrophobic compounds, improving their solubility and stability. Furthermore, they have a lipid layer similar to that of cell membranes, which makes them biocompatible and gives them good affinity with the skin, facilitating their penetration. Therefore, these systems are used for the transport of active ingredients through the skin, improving their effectiveness. Additionally, liposomes are composed of phospholipids, whose properties are also useful in the treatment of skin pathologies: epithelializing (repairing the damaged stratum corneum, improving the skin barrier function), anti-inflammatory, bactericidal, sebum-regulating, and whitening (evening skin tone). Furthermore, ligands with affinity for a specific cell type associated with a particular pathology can be conjugated to the outer surface of liposomes. This allows liposomes loaded with active ingredients effective against that pathology to be targeted precisely to the affected areas, thus increasing the efficacy and specificity of the treatment.

In this case, a peptide with affinity for the chemokine receptor 3 (CXCR3) was chosen as the ligand. Chemokines are a group of low molecular weight polypeptides containing 60-100 amino acids that, together with their receptors, regulate inflammatory processes. The chemokine ligand 10 (CXCL10) is secreted by multiple cell types, including lymphocytes, monocytes, keratinocytes, fibroblasts, and endothelial cells in response to interferon (IFN)-γ and TNFα. CXCL10 has angiostatic properties and recruits leukocytes such as T cells, eosinophils, monocytes, and NK cells to sites of inflammation through interaction with its receptor CXCR3. This receptor is expressed in various cell types, but preferentially in monocytes, Th1 T cells, CD8 T cells, NKT cells, NK cells, dendritic cells, and some cancer cells.

Vitiligo is a skin pigmentation disorder that affects approximately 1-2% of the population, with no predilection for age, sex, or race. It is characterized by the appearance of achromic macules, whose typical histopathological substrate is the absence of identifiable melanocytes. Several factors contribute to the development of vitiligo's pathophysiology, including genetic, environmental, and immunological factors. The interaction between these factors culminates in autoimmunity as the main factor in the pathogenesis of the disease, exerted through autoreactive, melanocyte-specific, cytotoxic CD8 T lymphocytes guided by IFNγ. Vitiligo is, therefore, an autoimmune disease. Autoimmune diseases are those in which the immune system recognizes its own structures as foreign, causing their destruction and resulting in disease. The initial triggering events that lead to immune system activation in vitiligo arise from a combination of intrinsic defects in melanocytes and exposure to specific environmental factors, such as ultraviolet light or chemicals, that increase oxidative stress in these cells. In the present invention, the CXCR3B receptor was chosen as the target because it has been shown that, in skin with vitiligo, the expression of the CXCR3B receptor is increased in both immune cells and melanocytes.

On the other hand, psoriasis is a chronic disease characterized by reddish, patchy plaques on the skin that are inflamed and scaly. This is due to the hyperproliferation of keratinocytes, leading to high epidermal turnover, and the infiltration of immune cells into the dermis and epidermis. Individuals with this disease, who represent 1-5% of the world's population, suffer from symptoms such as itching and pain, as well as skin lesions, especially in exposed areas like the face, hands, and nails, which can significantly impact the patient's quality of life. Furthermore, it often coexists with comorbidities such as psoriatic arthritis, uveitis, psychiatric disorders, metabolic disorders, and cardiovascular diseases. It has an unknown etiology, is incurable, and is relapsing, presenting either in flare-ups or constantly due to triggering factors. Because epidermal changes are so prominent, psoriasis was assumed for years to be a disease of keratinocytes. However, psoriasis is actually an autoimmune disease mediated by T lymphocytes. In this disease, chemokines are involved in both the recruitment and activation of T lymphocytes, macrophages, and neutrophils at the site of psoriatic inflammation. Specifically, CXCR3 and its associated chemokine ligands constitute a very important chemotactic response to interferon-mediated inflammation. Therefore, cells of the immune system (epidermal T cells, plasmacytoid dendritic cells, and NK cells) express CXCR3.

In summary, in skin affected by vitiligo, the CXCR3B receptor is overexpressed in melanocytes, while in psoriasis, immune system cells, such as epidermal T cells, express CXCR3. Consequently, the liposomes of the present invention, functionalized with a specific ligand for this receptor, will act specifically on the cells affected by vitiligo and psoriasis, releasing the encapsulated active ingredients there, and not in adjacent cells. This will be an effective treatment with cell specificity and a low risk of long-term adverse effects.

Finally, to achieve this affinity for the CXCR3B receptor, a fragment of the chemokine CXCL10, a ligand for this receptor, was chosen as the functionalizing peptide. The specific amino acid sequence is CPESKAIKNLLKAVSKEMSKRSP (SEC ID NO 1). This sequence is partially described in US patent 2013053319 (A1) as PESKAIKNLLKAVSKEMSKRSP (SEC ID NO 2), differing from the sequence of the present invention by the incorporation of a cysteine at the N-terminus. The peptide binds covalently to the liposomes once formed by a click reaction between maleimide groups exposed on the exterior of the liposome and the thiol group at the end of the peptide. To facilitate the subsequent binding of the peptide to its target receptor, the maleimide-containing phospholipids have a polyethylene glycol spacer group between the phospholipid and the maleimide.

### Preferred implementation

In a preferred embodiment, the functionalized liposomes for obtaining cosmetic or pharmaceutical compositions comprise:
a) at least one peptide with the sequence CPESKAIKNLLKAVSKEMSKRSP SEC ID NO: 1 linked at its N-terminus to
b) a liposome comprising maleimide (Mal) groups on its exterior due to the incorporation of DSPE-PEG2000-Mal in the preparation of said system, and containing
c) at least one pharmaceutical or cosmetic active ingredient for the treatment of psoriasis and/or vitiligo

Functionalized liposomes contain the peptide with the sequence SEC ID NO: 1, which is identical to the peptide with the sequence SEC ID NO: 2, except for the presence at the beginning of the sequence of a cysteine residue necessary for its anchoring to the maleimide on the exterior of the liposome.

Functionalized liposomes may have a peptide anchored to the liposome that is different from the one with the sequence SEC ID NO: 1 but whose sequence is identical to 80% of the amino acids from position 2 to 23 of the sequence SEC ID NO: 1.

From these liposomes, a pharmaceutical or cosmetic composition for topical use is prepared, comprising the functionalized liposomes described above as the active ingredient, in combination with one or more pharmaceutically or cosmetically acceptable excipients or adjuvants, for topical use in the treatment of psoriasis and vitiligo.

An example of a pharmaceutical or cosmetic composition containing these functionalized liposomes, with the weight percentages of its components, would be the following:
Water: 75-100%
Phosphatidylcholine: 5-10%
DSPE-PEG-Mal: 0.1-2%
Alcohol: 1-5%
Polysorbate 20: 1-5%
Phenoxyethanol: 0.1-1%
Glycerin: 0.1-1%
Peptide: 0.1-4%
Active Ingredient: 0-5%

Having sufficiently described the nature of the invention, as well as an example of a preferred embodiment, it is noted that the described components may be modified, provided that this does not alter the essential characteristics of the invention claimed below.

## Claims

1. Functionalized liposomes for obtaining cosmetic or pharmaceutical compositions comprising:
a) at least one peptide with the sequence SEC ID NO: 1 linked at the N-terminus to
b) a liposome containing
c) at least one active pharmaceutical or cosmetic ingredient

2. Functionalized liposomes according to claim 1, wherein one of the liposome components is DSPE-PEG-Mal, such that the peptide is covalently linked to maleimide (Mal) groups located on the exterior of the liposome.

3. Functionalized liposomes according to claim 1, wherein the peptide with sequence SEC ID NO: 1 is identical to the peptide with sequence SEC ID NO: 2 except for the presence at the beginning of the sequence of a cysteine residue necessary for its anchoring to the exterior of the liposome.

4. Functionalized liposomes according to claims 1 and 3, wherein the peptide anchored to the liposome is different from that of sequence SEC ID NO: 1 but shares the same sequence as 80% of the amino acids from position 2 to 23 of sequence SEC ID NO: 1.

5. A pharmaceutical or cosmetic composition for topical use comprising the functionalized liposomes, as defined in claims 1-4, as an active ingredient, in combination with one or more pharmaceutically or cosmetically acceptable excipients or adjuvants.

6. Use of the pharmaceutical or cosmetic composition of claim 5 for obtaining cosmetic or pharmaceutical compositions for the treatment of psoriasis and vitiligo.
